Europäisches Patentamt

European Patent Office

Office européen des brevets

(11) Publication number: **0 049 464**
**B1**

## (12) EUROPEAN PATENT SPECIFICATION

(45) Date of publication of patent specification: **06.02.85**

(21) Application number: **81107746.0**

(22) Date of filing: **29.09.81**

(51) Int. Cl.⁴: **A 61 B 6/02**

(54) **Apparatus for collecting X-ray absorption data in a computerized tomographic apparatus.**

(30) Priority: **08.10.80 JP 141016/80**

(43) Date of publication of application:
**14.04.82 Bulletin 82/15**

(45) Publication of the grant of the patent:
**06.02.85 Bulletin 85/06**

(84) Designated Contracting States:
**DE FR GB NL**

(56) References cited:
**DE-A-2 950 780**
**US-A-3 971 948**
**US-A-4 029 963**
**US-A-4 149 081**

(73) Proprietor: **Kabushiki Kaisha Toshiba**
**72, Horikawa-cho Saiwai-ku**
**Kawasaki-shi Kanagawa-ken 210 (JP)**

(72) Inventor: **Yamagishi, Yoshio**
**385-44, Takeshita-cho**
**Utsunomiya-shi Tochigi-ken (JP)**

(74) Representative: **Patentanwälte Henkel,**
**Pfenning, Feiler, Hänzel & Meinig**
**Möhlstrasse 37**
**D-8000 München 80 (DE)**

Courier Press, Leamington Spa, England.

## Description

The invention relates to an apparatus for collecting X-ray absorption data of an object in a computerized tomographic apparatus, comprising an X-ray source for producing two different energies of X-radiation and a detector disposed opposite to said X-ray source, and means for causing relative translation of said X-ray source and detector together with respect to said object and perpendicular to an axis parallel to the line joining the source and the detector such that said X-ray source and detector remain substantially in a plane and for collecting radiation absorption data of said object to reconstruct an image representative of the distribution of said radiation absorption in said plane.

Among X-ray tomographic apparatus, there are computerized tomographic scanners (hereinafter referred to as CT scanner).

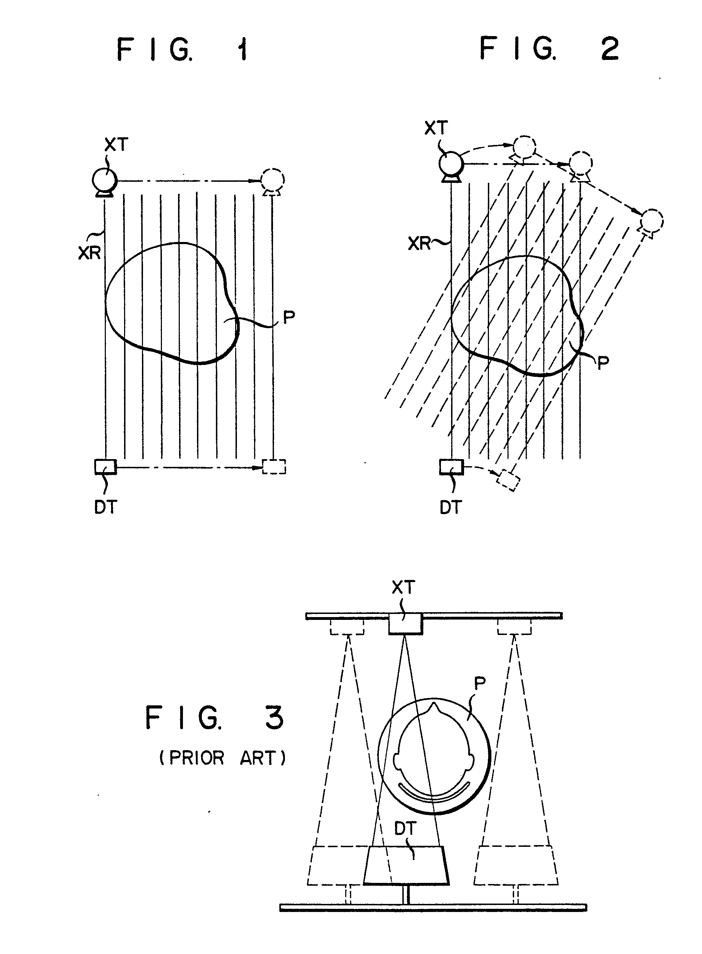

One such CT scanner adopts a traverse and round system also called first generation. Fig. 1 shows this first generation. In this system, an X-ray tube XT and an X-ray detector DT are disposed to face each other via a patient's body P, and they are traversed together in the direction of arrows in Fig. 1, while the X-ray tube emitting X-rays along a plane section of the body P. The X-ray tube XT and the X-ray detector DT being so traversed, a radiation path XR is obtained which is parallel to the plane section of the body P. After one cycle of such scanning is ended, the angle of incidence of X-radiation on the plane section of the body P is changed. The linear motion and rotational motion are alternately repeated to collect X-ray absorption data usually over an angle range of 180 degrees. The reconstitution of every point of the plane section of the body P is possible by permitting X-rays to be incident on the plane section substantially in all directions or angular aspects.

The X-ray absorption data obtained at the X-ray detector DT is analyzed in an electronic computor to calculate the X-ray absorption factor of individual points of the plane section. A tomographic image is reconstituted through the analysis of each point of the body's plane section on the basis of the determined X-ray absorption factor and with a graduation degree amounting to more than 2,000 steps. In addition to the first generation, there is another traverse and round system CT scanner called a second generation. As shown in Fig. 3, the second generation uses as the X-ray source an X-ray tube XT which generates X-rays in the form of sector having a small angle. An X-ray detector DT is disposed to face the X-ray tube XT via the body P mentioned above. The X-ray detector DT has a plurality of X-ray detecting elements arranged side by side. For the collection of X-ray absorption data, the X-ray tube XT and X-ray detector DT are traversed along a plane section of the body P and, after the completion of traverse scanning in each cycle, turned round by a predetermined angle.

As has been shown, either in the first or second generation the reconstitution of the final image involves image formation through conversion of the value of X-ray absorption that is determined by the structure of the inspected body into a corresponding shade signal. The X-ray absorption factor, i.e., the value of each piccell constituting the image, is referred to as CT value. This CT value is linearly related to the X-ray absorption factor and is called Hounsfiled number after its inventor's name. This value corresponds to an absorption factor value based on an X-ray energy of 73 keV (hereinafter referred to as $\mu$ value). The reconstitution of the final image is effected after deriving the CT value. However, the CT value is not an absolutely definite value for the following reasons. The X-radiation from the commercially available CT scanners usually does not consist of a single wavelength at the aforementioned reference energy level of 73 keV but has a spectral distribution. In addition, the X-ray absorption factor M of a predetermined structure of the body is not fixed because of variations of the proportions of the photoelectric effect and Compton effect. More specifically, the X-ray absorption in the neighborhood of a predetermined structure is surrounded by bones, and an energy spectrum shift, if any, causes a deviation of the CT value. The X-ray energy distribution which has the most significant influence upon the X-ray absorption value, can be known by varying the tube voltage of the X-ray tube.

Most of the existing CT scanners use only a single fixed tube voltage to achieve the linear scanning of a plane section of the body. It is therefore necessary to scan the same plane section two or more times, moving the X-ray tube and X-ray detector repeatedly and applying a different voltage on the X-ray tube during each linear scanning. This increases the exposure dose of X-rays and requires a longer time, inevitably causing the patient much trouble. In view of this the existing CT scanners cannot be said to be so practical.

It has been contemplated to provide a process of compensation for the shift of the CT value during the calculation for the image reconstitution. However, no correction method that is effective with any body and also for any inspecting condition has been found.

From US—A—4 029 963 an X-ray spectral decomposition imaging system is known which uses an X-ray beam in low and high energy regions. The beam has a fan configuration and is interrupted by a rotating filter wheel containing a low-energy filter and a high-energy filter, so that alternatively low-energy and high-energy beams can pass the wheel. The transmitted beam simultaneously exposes the whole object; therefore no relative translation and no relative rotation of the radiation source and the radiation detector with respect to the object are performed.

US—A—3 971 948 shows an X-ray diagnostic apparatus with two measuring arrangements, each consisting of an X-ray tube and a radiation receiver. The X-ray tubes generate radiation having a different value of energy. The measuring arrangements are parallelly disposed with respect to each other and simultaneously moved in relation to the object to be scanned.

Finally, US—A—4 149 081 discloses an instrument for reconstructing computerized tomograms utilizing penetrating radiation. The tomograms are produced with radiation of two-level energy. The measurements can be performed either by means of different peak energy setting of the source, the use of a source input filter or the utilization of detectors of different efficiencies of different response ranges. However, here also the whole object is exposed simultaneously. Therefore, no relative translation of the measuring arrangement with respect to the object takes place. Only a relative rotation is feasable.

An object of this invention is to provide an apparatus for collecting X-ray absorption data in an X-ray tomographic apparatus, in which the collection of X-ray absorption data with respect to a plane section of the body is effected on the basis of different tube voltages using a single measuring arrangement which is movable in relative translation with respect to the body to be scanned.

The apparatus for collecting radiation absorption data of an object in a computerized tomographic apparatus, as mentioned in the beginning, is characterized in that said means causes the relative translation to be performed in a first direction and a second direction opposite to said first direction and switching means are provided to select one of said two different energies of said X-ray source in dependence on said first and second directions of said relative translation such that two different absorption data are provided respectively for the first and opposite direction of translation and after each said relative translation in the first and opposite directions said means causes the direction of translation to be rotated through an angle about said axis so that the relative translation can be repeated in several directions about said axis.

With the above apparatus for collecting X-ray absorption data in an X-ray computor tomographic apparatus according to the invention, two different kinds of X-ray absorption data with respect to a given plane section of the body which are respectively based on the two different tube voltages, can be collected in a short period of time, and a highly accurate image with the CT value close to the characteristics of the structure of the body can be obtained from three different kinds of X-ray absorption data, namely two based upon the two different tube voltage and the last one as the difference between these two different data.

This invention can be more fully understood from the following detailed description when taken in conjunction with the accompanying drawings, in which:

Fig. 1 is a view useful to explain the scanning operation of a prior art first generation CT scanner;

Fig. 2 is a view useful to explain the rotational operation of the prior art CT scanner shown in Fig. 1;

Fig. 3 is a view useful to explain the traverse operation of a prior art second generation CT scanner;

Fig. 4 is a view showing the orbit of motion of X-ray tube and useful to illustrate the method of collecting X-ray absorption data according to the invention;

Fig. 5 is a block diagram, partly in schematic, outlining an X-ray absorption data collection apparatus in an X-ray tomographic apparatus according to the invention; and

Fig. 6 is a block diagram illustrating the function of a computor shown in Fig. 5.

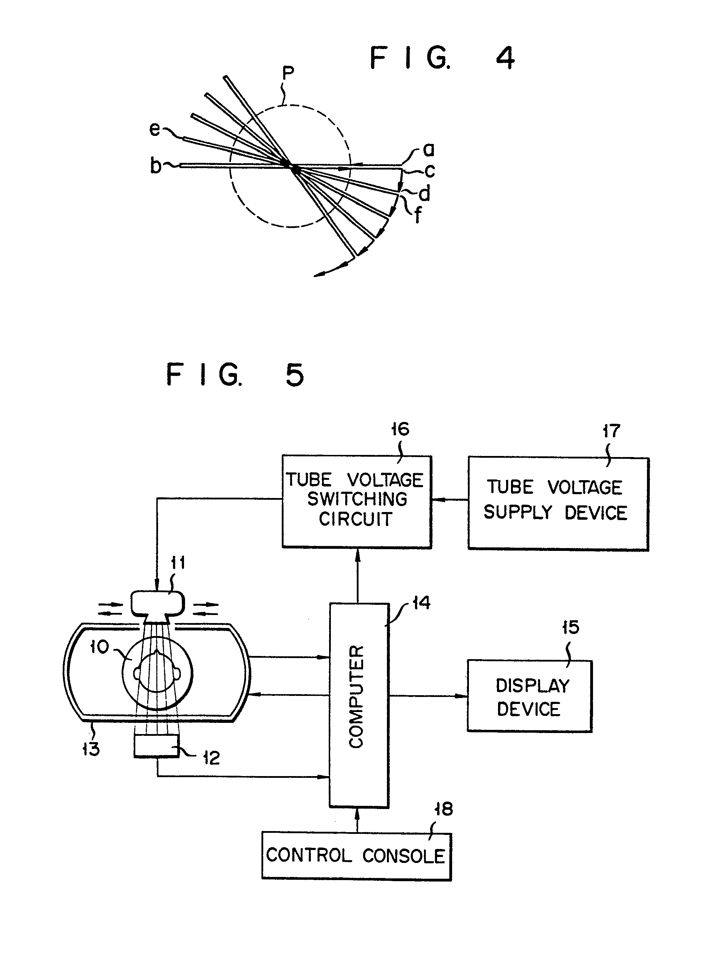

Now, an embodiment of the invention will be described with reference to Figs. 4, 5 and 6.

The invention is applied to a first generation and a second generation CT scanner. Fig. 4 shows the orbit of movement of an X-ray tube and an X-ray detector disposed to face each other via a body.

The X-ray tube and X-ray detector which are located at a position a is linearly traversed forward to scan the body P with the X-ray tube emitting X-rays on the basis of a predetermined tube voltage, for instance 100 kV. In the backward or return traverse, the X-ray detector detects X-ray absorption data in a plane section of the body P in a given angular aspect. When the X-ray tube reaches a point b, its voltage is switched to, for instance, 140 kV. Then, the X-ray tube and X-ray detector return to the point c. During their return traverse, X-ray absorption data is obtained on the basis of a different tube voltage from that during the forward traverse. Thus, two different kinds of X-ray absorption data are obtained with respect to the same plane section. Then, the X-ray tube and X-ray detector at the point c are rotated by a predetermined angle to a point d. When the point d is reached, the X-ray tube voltage is again switched to the aforementioned predetermined voltage again, and during the subsequent forward traverse up to the point c X-ray absorption data is detected with respect to the X-rays that are emitted from the X-ray tube toward the body P on the basis of the aforementioned predetermined tube voltage. Upon reaching of the point c, the tube voltage is switched once again, and X-ray absorption data is detected on the basis of the different tube voltage from that during the forward traverse until the X-ray tube and X-ray detector arrive at a point f. The sequence of linear motion and rotational motion as mentioned are repeated, and the detection of X-ray absorption data with respect to a given plane section is ended with the completion of 180-degree rotation.

The detection or collection of data on the basis of two different tube voltages, one during the forward traverse and the other during the return traverse in the translational reciprocal scanning, permits obtaining three different images, namely two images based on the respective tube voltages during the forward and return traverses and an image obtained by correction with the difference between the X-ray absorptions based on the two different tube voltages. Thus, an image having a CT value close to the structure of the body can be obtained. Particularly, it is possible to make analysis of the X-rays' electron density and the structural element composition of the body from the aforementioned X-ray absorption difference data.

Now, an X-ray absorption data collection apparatus according to the invention will be described with reference to Fig. 5.

An X-ray tube 11 and an X-ray detector 12 are supported by a frame 13 such that they face each other via a body 10. The X-ray tube 11 and X-ray detector 12 are capable of both linear motion and rotational motion with respect to the body 10.

A start instruction to start the scanning of the X-ray tube and X-ray detector 12, a scanning speed switching instruction and/or a slice width switching instruction are supplied as control instruction signal A from a computor 14 to the CT scanner.

The aforementioned two different X-ray absorption data based on the different tube voltages, detected by the X-ray detector 12, are coupled as data detection signal B to the computor 14. In the computor 14, the data detection signal B is analyzed for display of reconstruction image on a display unit 15 at a graduation degree corresponding to X-ray absorption factor at each point of the plane section.

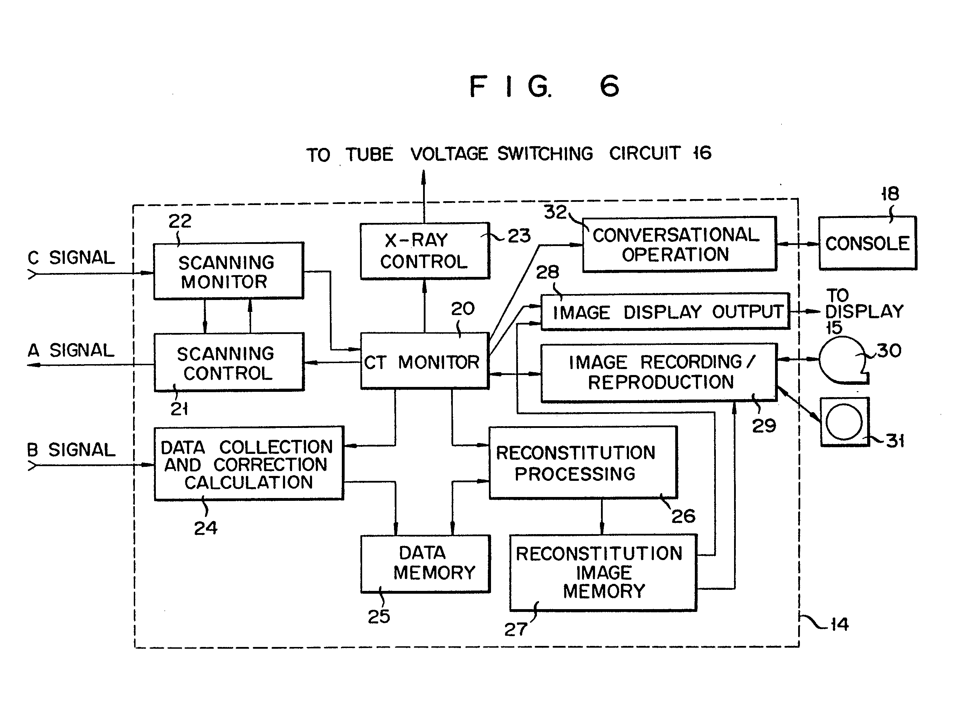

A signal C which indicates whether the X-ray tube 11 and X-ray detector 12 are in their forward or return travel is also coupled to the computor 14. A tube voltage supply device 17 is connected through a tube voltage switching circuit 17 to the X-ray tube 11. The tube voltage switching circuit 16 is connected to the computor 14. When the computor 14 detects the end of the forward or return traverse from the aforementioned signal C from the CT scanner, it supplied a control signal to the tube voltage switching circuit 16 to switch the tube voltage supplied from the tube voltage supply device 17 to the X-ray tube 11. The setting of the computor 14 in accordance with the system condition of the CT scanner is done from a control console 18. The function of the computor 14 will now be described with reference to a block diagram of Fig. 6.

A scanning control circuit 21 generates the instruction signal A supplied to the CT scanner according to an instruction signal from a CT monitor 20. A scanning monitor 22 detects the aforementioned signal C from the CT scanner, indicating the state of motion of the X-ray tube 11 and X-ray detector 12, and reports the detection to the CT monitor 20. After confirming the scanning state of the CT scanner, the CT monitor 20 gives an instruction to an X-ray control circuit 23 to let the control circuit 23 supply a control signal to the tube voltage switching circuit 16 mentioned above according to the detected scanning state. A data collection and correction calculation circuit 24 collects the X-ray detection data signal B corresponding to the affected part of the body 10, detected by the X-ray detector 12 mentioned above, and effects correction calculation with the detection data signal B in accordance with an instruction supplied from the CT monitor 20. The result of calculation is stored in a data memory 25. A reconstitution processing circuit 26 receives the stored data output from the data memory 25 and an instruction output from the CT monitor 20 and reconstitute the X-ray detection data. The reconstitution data is stored in a reconstitution image memory 27. An image display output circuit 28 receives the stored data from the reconstitution image memory 27 and an instruction signal from the CT monitor 20 and supplies an image display output to the image display unit 15 mentioned above for display. An image recording/reproducing circuit 29 receives the stored data from the reconstitution image memory 27 and an instruction signal from the CT monitor 20 and can let the received reconstitution image be stored on a magnetic tape 30 or on a flopply disc 31.

The CT monitor 20 is connected through a conversation operation circuit 32 to the console 18 mentioned above. The conversation operation circuit 32 can set the operation procedure depending upon the condition of the CT system under the control of an instruction signal from the control console 18.

## Claims

1. Apparatus for collecting X-ray absorption data of an object 10 in a computerized tomographic apparatus, comprising an X-ray source (11) for producing two different energies of X-radiation and a detector (12) disposed opposite to said X-ray source, and means (13, 14) for causing relative translation of said X-ray source (11) and detector (12) together with respect to said object (10) and perpendicular to an axis parallel to the line joining the source and the detector such that said X-ray source (11) and detector (12) remain substantially in a plane and for collecting radiation absorption data of said object to reconstruct an image representative of the distribution of said radiation absorption in said plane, characterized in that said means causes the relative translation to be performed in a first direction and a second direction opposite to said first direction and switching means are provided to select one of said two different energies of said X-ray source in dependence on said first and second directions of said relative translation such that two different absorption data are provided respectively for the first and opposite direction of translation and after each said relative translation in the first and opposite directions said means causes the direction of translation to be rotated through an angle about said axis so that the relative translation can be repeated in several directions about said axis.

2. Apparatus according to claim 1, characterized in that said X-ray source comprises a tube voltage supply device (17) for producing two different voltages and a tube voltage switching circuit (18) for connecting said X-ray source (11) with one of said two different voltages.

## Patentansprüche

1. Gerät zum Erfassen von Röntgenstrahl-Absorptions-werten eines Objekts (10) in einem rechnergesteuerten Tomographie-Gerät umfassend eine Röntgenstrahlungsquelle (11) zur Lieferung zweier verschiedener Energien der Röntgenstrahlung und einen der Röntgenstrahlungsquelle gegenüberstehend angeordneten Detektor (12) sowie eine Einrichtung (13, 14) zur Hervorbringung einer Relativverschiebung der Röntgenstrahlungsquelle (11) zusammen mit dem Detektor (12) in bezug auf das Objekt (10) und senkrecht zu einer Achse, die parallel zu der die Röntgenstrahlungsquelle und den Detektor verbindenden Linie verläuft, so daß Röntgenstrahlungsquelle (11) und Detektor (12) praktisch in einer Ebene verbleiben, und zum Erfassen oder Sammeln von Strahlungsabsorptionswerten des Objekts zwecks Rekonstruktion eines für die Verteilung der Strahlungsabsorption in der genannten Ebene repräsentativen Bilds, dadurch gekennzeichnet, daß dei genannte Einrichtung die Relativ-verschiebung in einer ersten Richtung und in einer zweiten, zur ersten Richtung entgegengesetzten Richtung hervorbringt umd Umschaltmittel zum Wählen einer der beiden verschiedenen Energien der Röntgenstrahlungsquelle in Abhängigkeit von der ersten und zweiten Richtung der Relativverschiebung vorgesehen sind, so daß zwei verschiedene Absorptionswerte für die erste bzw. die entgegengesetzte Verschiebungsrichtung geliefert werden, und die genannte Einrichtung nach jeder Relativverschiebung in der ersten und der entgegengesetzten Richtung eine Drehung der Verschiebungsrichtung über einen Winkel um die genannte Achse hervorbringt, so daß die Relativverschiebung in verschiedenen Richtungen um diese Achse herum wiederholbar ist.

2. Gerät nach Anspruch 1, dadurch gekennzeichnet, daß die Röntgenstrahlungsquelle eine Röhrenspannungsspeisevorrichtung (17) zur Lieferung zweier verschiedener Spannungen und eine Röhrenspannungsumschaltschaltung (18) zum Anschalten der Röntgenstrahlungsquelle (11) an (jeweils) eine der beiden verschiedenen Spannungen aufweist.

## Revendications

1. Appareil de prise de données d'absorption de rayons X d'un objet (10) dans un appareil de tomographie à ordinateur, comprenant une source de rayons X (11) pour produire deux énergies différentes de radiations X et une détecteur (12) disposé à l'opposé de la source de rayons X, et des moyens (13, 14) pour obtenir une translation relative de l'ensemble source de rayons X (11)-détecteur (12) par rapport à l'objet (10) dans une direction perpendiculaire à un axe parallèle à la ligne joignant la source au détecteur de sorte que la source de rayons X (11) et le détecteur (12) restent essentiellement dans un plan et pour recueillir des données d'absorption de radiations par l'objet en vue de reconstruire une image représentant la répartition de l'absorption de radiations dans ledit plan, caractérisé en ce que lesdits moyens sont agencés pour que la translation relative soit effectuée dans une première direction et dans une seconde direction opposée à la première direction et en ce qu'il comprend des moyens de commutation pour sélectionner une des deux énergies différentes de la source de rayons X en fonction desdites première et seconde directions de la translation relative de sorte que deux données d'absorption différentes sont respectivement obtenues pour la première direction et la direction opposée de translation, et, après chaque translation relative dans la première direction et dans la direction opposée, en ce que lesdits moyens font tourner la direction de translation d'un certain angle autour dudit axe de sorte que la translation relative peut être répétée dans plusieurs directions autour dudit axe.

2. Appareil selon la revendication 1, caractérisé en ce que ladite source de rayons X comprend un dispositif d'alimentation en tension de tube (17) pour produire deux tensions différentes et une circuit de commutation de tension de tube (18) pour appliquer à la source de rayons X (11) une des deux tensions différentes.

FIG. 1

FIG. 2

FIG. 3

(PRIOR ART)

1

# F I G. 4

# F I G. 5

# F I G. 6

0 049 464